# EUROPEAN PATENT SPECIFICATION

(11) **EP 3 417 690 B1**
(45) Date of publication and mention of the grant of the patent: **25.11.2020**
(21) Application number: 18178272.3
(22) Date of filing: 18.06.2018
(51) Int. Cl.: A01C 21/00, A01B 79/00, G01N 33/00, G01N 33/24

(54) **A SELF-PROPELLED APPARATUS FOR OPTIMALLY ANALYSING AND MANAGING FIELDS INTENDED FOR AGRICULTURAL CULTIVATION**
SELBSTANGETRIEBENE VORRICHTUNG ZUR OPTIMALEN ANALYSE UND BEWIRTSCHAFTUNG VON FELDERN FÜR DEN LANDWIRTSCHAFTLICHEN ANBAU
APPAREIL AUTOPROPULSÉ POUR L'ANALYSE ET LA GESTION OPTIMALES DES CHAMPS DESTINÉS À LA CULTURE AGRICOLE

(30) Priority: 19.06.2017 IT 201700067764
(43) Date of publication of application: 26.12.2018
(73) Proprietor: DINAMICA GENERALE S.p.A, 46025 MN Poggio Rusco (IT)
(72) Inventor: BARBI, Alberto, 46025 Poggio Rusco (IT); GHIRALDI, Andrea, 46025 Poggio Rusco (IT); GHIRALDI, Anna, 46025 Poggio Rusco (IT)
(74) Representative: Gagliardelli, Fabrizio

(56) References cited:
- WO-A1-91/04484
- WO-A1-2016/175094
- WO-A1-2017/004074
- US-A- 5 467 271
- US-A1- 2016 050 840
- US-A1- 2017 127 606

## Description

The present invention concerns a self-propelled apparatus for optimally analysing and managing fields intended for agricultural cultivation.

In detail, the invention relates in particular to a self-propelled apparatus designed to perform analysis based both on samples and remote measurements, by integrating the results for the purposes of precision agriculture.

Agricultural machines are known, such as tractors, which are equipped with analysis instruments for monitoring the chemical/physical conditions of cultivated soils.

In practice, such instruments are mounted on board the agricultural vehicle, for example, they may be towed on a trailer by the vehicle or be positioned on a device located at the front of the vehicle itself.

The data acquired by such instruments is used to regulate the cultivation procedures, for example irrigation, fertilisation, disinfestation or the like, according to the different conditions of the various areas of the fields.

The need to collect and accurately analyse such data is increasingly pressing, due to the process of desertification which is progressively reducing cultivable areas in many parts of the world.

Since the available area of cultivable land is shrinking while human and livestock populations are growing, there is a need to increase the output per hectare of cultivation and hence to increase its efficiency, preferably using ecologically sound techniques.

Known solutions have not proved able to fully satisfy this need, since they do not permit real time acquisition and management of a sufficiently wide range of data to enable efficient analysis of the most probable causes of damage to vegetation or efficient characterisation of the soil prior to sowing.

In both cases, the collection and processing of such data provides the basis for obtaining the information required to optimally manage treatments.

In detail, the treatments are differentiated per section of the field based on actual needs which are always at the basis of precision agriculture or precision farming.

Furthermore, the known solutions do not enable real time analysis of the collected data, which would allow prompt action to be taken in the interests of preventing the worsening of the damage.

US2016050840 discloses a drone for agricultural monitoring which can comprise cameras for performing analysis while flying. In detail, the drone comprises a camera system which might include a still photo camera, a video camera, a thermal imaging camera, and/or a near infrared camera for capturing normalized difference vegetation index images. WO2017004074 also discloses a drone to be used in the agricultural field, which can be equipped with sensors to detect electromagnetic radiation and also discloses the idea of bringing these sensors close to specific soil samples.

The technical task of the present invention is thus to propose a self-propelled apparatus for analysing and managing open field cultivation which satisfies the above requirements.

The technical task is achieved by the machine provided in accordance with claim 1.

Further characteristics and advantages of the present invention will become more apparent from the indicative and thus non-limiting description of a preferred, but not exclusive, embodiment of an apparatus, as illustrated in the accompanying drawings, wherein:

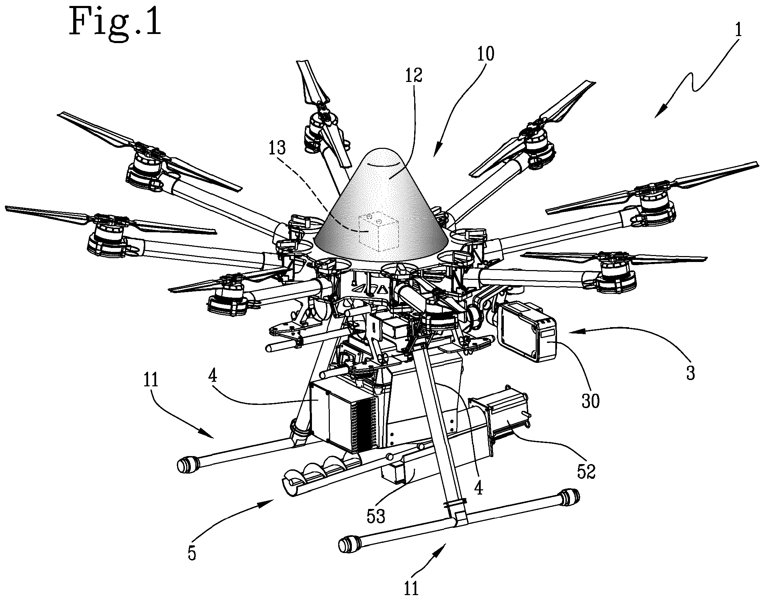

- figure 1 is an axonometric view of the apparatus of the invention, in an embodiment which envisages the use of a drone;

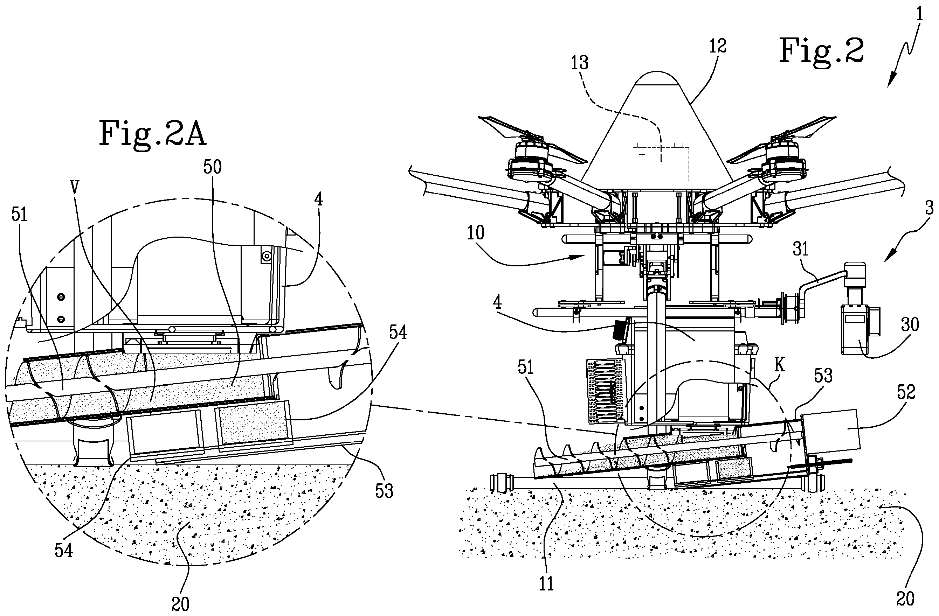

- figure 2 is a lateral view, partly in section, of the drone of the preceding figure;
- figure 2A is an enlarged view of detail K of the preceding figure;

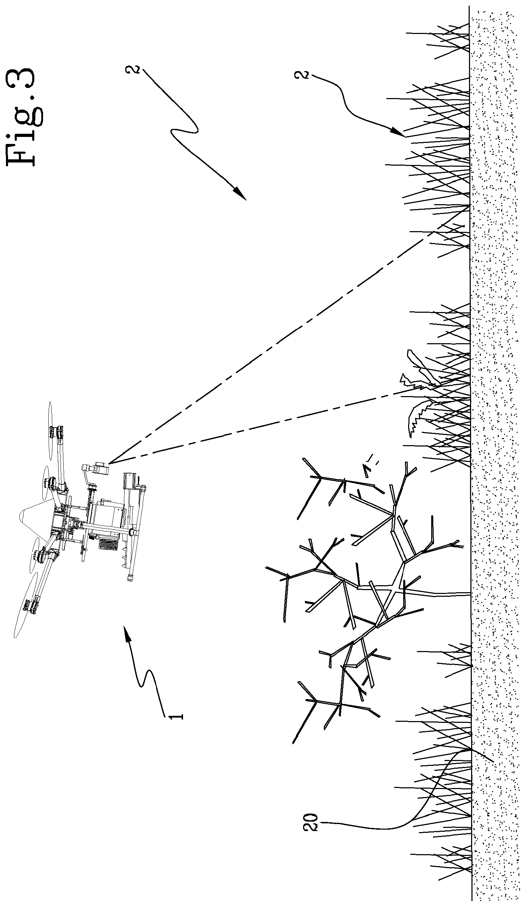

- figure 3 is a schematic representation of the drone of the invention, flying over a field;

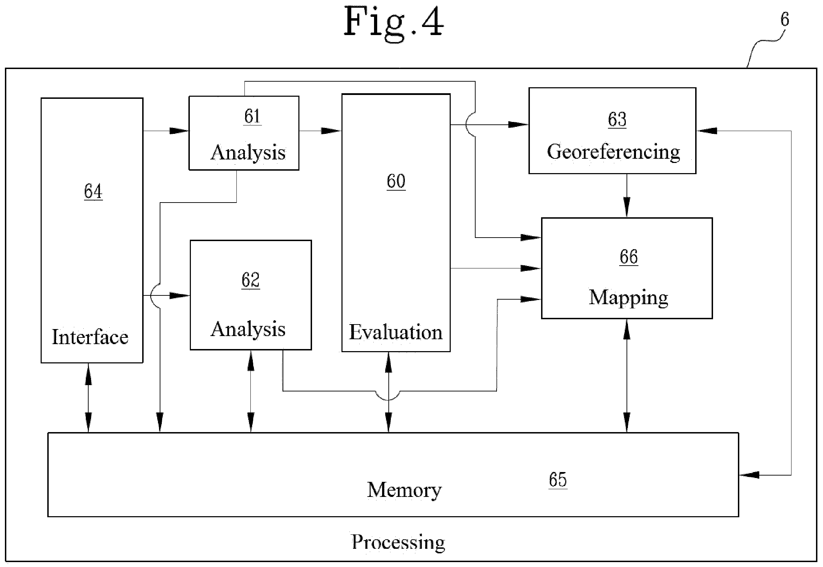

- figure 4 shows a diagram representing the processing unit of the invention, articulated into its operating and memory modules.

With reference to the cited figures, 1 denotes a self-propelled apparatus for analysing and managing a field 2, either cultivated or intended to be cultivated.

In the example illustrated in the accompanying drawings, the proposed apparatus 1 includes a drone 10, specifically an octocopter; however, the invention may envisage the use of different means, for instance other aircrafts as well as ground vehicles, whether radio-controlled, automatic or controlled by a driver, such as tractors or the like.

Hereinafter, without loss of generality, reference will be made to the exemplary but not exhaustive case in which the apparatus 1 of the invention comprises a drone 10.

The proposed apparatus 1 comprises a first remote detection device 3 adapted for acquiring electromagnetic radiation emitted by vegetation 21 and/or soil 20 of the field 2 and adapted for producing first measurement signals representative of the acquisitions performed.

More precisely, such electromagnetic radiation may cover a range of frequencies which includes the visible, infrared and/or ultraviolet.

In detail, the first detection device 3 may comprise a camera 30, preferably of the hyperspectral type.

For example, the camera 30 (or other optical detection device) may be mounted on board the drone 10; in particular, the camera 30 may be supported by an articulated arm 31 mounted to the drone 10, controlled by servomechanisms or other means, to enable adjustment of the orientation of the camera 30 itself.

The apparatus 1 further includes a second detection device 4 for the spectroscopic analysis of the soil 20 which preferably includes one or more VIS - NIR 4 (Visible/Near Infrared) spectrometers, capable of analysing electromagnetic radiation in the visible or near infrared regions. The second device 4 is adapted for producing second measurement signals representative of the analysis performed.

In detail, the proposed apparatus 1 includes, mounted on board the drone 10, a sampling device 5 adapted for picking one or more samples 50 of soil.

According to the invention, as shown in the accompanying drawings, said device 5 includes a rotating picking auger 51, preferably rotationally driven by a motor 52, possibly electric, said auger 51 being predisposed for removing samples 50 from the soil 20.

Additionally, the sampling device 5 may comprise different picking elements, for example adapted to sample cores, or slat elevators, and so on.

More precisely, the auger 51 may be mounted inclined with respect to the central axis C of the drone 10 and arranged so that, relative to the supporting feet 11 of the drone 10, it has a free terminal end tilted downwards, to enable its penetration into the soil 20, thus enabling collection of the samples 50; this aspect will be discussed in greater detail hereinafter, upon explanation of the operation of the invention.

As can be seen in the accompanying drawings, the supporting feet 11 of the drone 10 may be realised with co-planar rectilinear rods joined to the support frame of the drone 10 by means of suitable arms.

Furthermore, the invention may envisage the use of an internal collection volume V, supplied by the auger 51, predisposed for receiving the collected samples 50 of soil.

In this case, the second detection device 4 is positioned so as to run spectrographic analysis of the samples 50 received in the collection volume V.

Said collection volume V may be afforded in a casing 53, passed through by the auger 51, which is faced onto by the NIR sensors via a suitable opening.

Furthermore, the casing 53 includes a lower opening, located below the collection volume V, predisposed to dump the samples after analysis. Furthermore, the sampling device may comprise one or more containers for the collected samples 50, housed with the option of moving underneath the collection volume V, for applications detailed below.

The proposed apparatus 1 may include a receiver for a satellite positioning system, for example of a GPS type, preferably located on board the drone 10, so as to check in real time the geographical coordinates thereof; precisely, the receiver is specifically but not exclusively predisposed for georeferencing the points of collection of the samples 50 of soil, as better illustrated below.

The invention also includes a processing unit 6 for analysing the measurements made by the two devices 3, 4 cited above, which may be located (partly or entirely) in a remote fixed central unit or (partly or entirely) on board the drone 10, in the second case it may be integrated into or coincide with the control unit of the drone 10 or it may be separate and distinct therefrom.

In the first case, the drone 10 represents the movable means of the apparatus 1 and the detection devices 3, 4 are connected to the processing unit 6 via radio frequency signal transmission means or similar telecommunications means; in the second case, the processing unit 6 may be adapted for receiving the first and second measurement signals directly, not mediated by radio signals.

More precisely, in a preferred embodiment of the invention, the processing unit 6 comprises two distinct devices, one of which is located on board the drone 10 and the other one remotely, for example a server in a control centre or "on the cloud".

The two devices may communicate via modem or other equivalent means adapted to the purpose.

In general, it is to be noted that, in the present disclosure, the processing unit 6 is presented divided into distinct functional modules for the sole purpose of describing the functions thereof in a clear and complete manner.

In practice, such processing unit 6 can be made up of a single electronic device, appropriately programmed to perform the functionalities described; the different modules can correspond to hardware entities and/or software routines that are part of the programmed device.

Alternatively or in addition, such functions may be performed by a plurality of electronic devices over which the aforesaid functional modules can be distributed.

In general, the processing unit 6 can use one or more microprocessors for performing the instructions contained in the memory modules and the aforesaid functional modules may also be distributed over a plurality of local or remote calculators based on the architecture of the network in which they reside.

According to an important aspect of the invention, the processing unit 6 includes a first analysis module 61 configured to determine first data representative of the vegetation 21 and/or the soil 20 subject to detection, calculated in relation to the first measurement signals, and a second analysis module 62 configured to determine second data representative of the soil 20 subject to detection, calculated in relation to the second measurement signals.

In detail, the first analysis module 61 may be configured to determine the said first data by means of image processing techniques for processing images acquired by the first device 3, i.e. using imaging techniques (also of a known type) and/or by means of thermographic analysis.

In practice, the invention provides a drone 10 (or other self-propelled vehicle) capable of acquiring both images from above of the fields 2 over which it is moving, with the camera 30, to monitor in particular the health of the vegetation 21 and/or the condition of the soil prior to sowing, and chemical/physical data about the soil 20.

The processing unit further comprises an evaluation module 60 configured to determine evaluation states of the soil and vegetation analysed, in accordance with the respective first and second data provided by the analysis modules 61, 62.

By "evaluation states" are meant characteristic states of the field (or a portion thereof) based on a judgement of value, which for example refers to the health of the crops or the nutritional richness of the soil, or yet other factors; other details on the types of evaluation states determined by the invention and on how they are determined will be provided in the following. Thanks to the analysis modules 61, 62 and the evaluation module 60, the checks of the state of the field 2 or, rather, the states of the various portions of the field 2, may be processed in real time, thus keeping the user up to date about the condition of the crops and enabling him to take prompt action to resolve deficiencies and abnormalities and to maximise the general productive efficiency of the field 2.

Preferably, by way of a non-exhaustive example, the evaluation module is configured to determine one or more of the following evaluation states based on the processing of the said first data: flourishing or unhealthy vegetation 21, dry or too wet vegetation 21, dry or wet soil 20, sandy or clay soil, plants 21 with hot or cold stems.

Furthermore, on the basis of the second data produced by the second module 62, the evaluation module 60 is preferably configured to determine, by way of a non-exhaustive example, one or more of the following second states of the soil 20: lack of nutrients including nitrogen, phosphorus, sodium, calcium, magnesium or iron, lack of oxygen, and excess of organic residues.

Before describing further preferred aspects of the proposed apparatus 1, its basic operation will be briefly described, with reference to the preferred embodiment shown in the accompanying drawings.

The field 2 is examined by having the drone 10 complete two missions.

During the first mission, the drone 10 of the invention flies over the field 2 to scan it, under the control of an operator or completely autonomously, and acquires images from above, and hence from a distance, with its camera 30.

Once the first mission has terminated, and the first measurement signals have been processed in order to yield in real time the said first data, the second mission of the drone 10 is specified, during which the drone 10 flies to specific areas of the field selected according to the first data, where it descends, lands to the gound, and then picks samples 50 of soil which are immediately analysed by the VIS - NIR 4 spectrometer.

In real time, the processing unit generates the second data mentioned above.

Note that, following the spectrometer analysis, in normal conditions, the sample picked by the auger 51 and conveyed into the collection volume V is dumped onto the ground via the opening mentioned above.

However, if the analysis of such sample detects anomalous values, the sample may be held within the casing 53, so that, on conclusion of the mission, it may be made available to the operator for further analysis to determine the cause of the anomalies.

In further detail, when an outlier sample is detected, one of the above mentioned containers 54 is moved under the collection volume V, so that the sample, rather than being dumped onto the ground, is received into the container 54.

The movement of the containers 54 is controlled handling equipment, known on their own, such as linear motors, pneumatic or hydraulic actuators, and so on.

In practice, the samples held on board the drone 10 are accumulated in a stack, i.e. in a sequence in which the position of each sample is known.

In detail, the processing unit 6 is configured to register in the memory module 65 the spectrum of each sample held on board the drone 10 along with its position in the said stack.

Hence, thanks to the invention, the user can acquire, for various sectors of the field 2, both data derived from the images acquired by the hyperspectral camera 30, relating above all to the condition of the vegetation 21, and data regarding the chemical/physical composition of the soil 20 on which the plants 21 are growing.

In this manner, for the various sectors of the field 2, the user has available a wide range of data, processed in real time, which enable him to diagnose any problems in a precise manner.

For example, should the apparatus 1 find that a particular sector of the field 2 has vegetation 21 which is unhealthy despite the soil 20 being rich in nutrients and oxygen, well hydrated and without excess organic residues, attention will be paid to exogenous causes, such as pollution by large industrial installations, lack of light, presence of infestation, etc.

In detail, the processing unit 6 may include a geo-referencing module 63 configured to uniquely associate coordinates to uniquely geo-reference the various sectors of the field 2 being analysed, and associate with them the first and second data provided by the analysis modules 61, 62 and the evaluation states calculated by the evaluation module 60, said coordinates being acquired by the above mentioned satellite receiver.

In practice, both the analyses based on the images acquired by the camera 30 and those based on the samples of soil are geo-referenced as the drone 10 flies over the cultivated field 2, and hence it is possible to uniquely associate the results of the analyses with the sectors to which they belong; this is the necessary condition for acquiring information which enables action to be taken with the tools of precision agriculture, based on so-called "prescription maps".

In this way, if for instance the field 2 comprises a sector which is poor in nitrogen and another which is very dry, the operator can compensate these problems in a targeted and efficient manner, with suitable prescription maps designed to control the fertilisation and irrigation processes, respectively.

In order to ensure proper division of the field 2 into sectors with different problems or requirements, it may be useful to provide the processing unit 6 with evaluation parameters which permit, for example, classification of the sectors themselves.

In particular, the evaluation parameters may be representative of upper and lower thresholds which segment the collected data into disjoint classes. In more general terms, the processing unit 6 may comprise or be connected to a user interface 64 configured to enable the operator to select or set first evaluation parameters representative of states of health of the vegetation 21 and/or of states of organic quality of the soil 20 and second evaluation parameters representative of states of chemical quality of the soil 20.

In this case, the evaluation module 60 is configured to determine the evaluation states according to the first and second evaluation parameters, respectively.

More precisely, the processing unit 6 of the invention employs regression algorithms evaluated on the processing of the images obtained by the camera and the processing of the spectra acquired by the spectrometer(s). All measured and geo-referenced data are transferred to the map-server service, assigning each sector of the field to a given class according to the characteristic threshold values associated with the parameters selected by the users.

The various operating modules of the processing unit 6 described above are connected to one or more memory modules 65, to enable recording of the qualitative states of the vegetation 21 and soil 20 of a field 2 examined by the apparatus 1 of the invention, divided by sectors.

The processing unit 6 may include a mapping module 66, connected to the memory module 65 and configured to produce a map of the field 2, i.e. a virtual representation thereof, in which for each sector of the field, on various layers, the evaluation states determined by means of the apparatus of the invention are mapped.

Furthermore, the map thus produced may include visual indices, for example composed of different colours, icons, digits or other graphic elements, representative of the states detected in the various sectors of the field 2 represented by the map.

Thanks to the information contained in the map, and to algorithms capable of optimising the various activities, the user may define the prescription maps: precision maps which specify different treatments for the various sectors of the field in order to optimise the treatment costs, minimise its environmental impact and maximise the harvest: in other words, to optimise the output of the resources employed.

In addition to the above, note that the operating logic of the preferred embodiment of the invention provides that input information be acquired from the cited hyperspectral camera, spectrophotometer/spectrometers and the GPS device, which is then transferred to the remote server.

Furthermore, the following functions are implemented in real time:
- calculation of a variety of indices (for example: NDVI (vegetation index); chlorophyll concentrations; thermal map or the like, based on known image analysis techniques, known on their own;
- association of the calculated indices to the GPS coordinates; and
- transmission of the geo-referenced information to a program adapted for providing a map-server service to generate the cited geo-referenced multilayer maps; in practice, to each sector of the field (identified by its GPS coordinates) are assigned as many layers as there are measured indices and a class is associated to each layer;
- on the basis of the geo-referenced maps constructed for the field, the operator selects the sectors of interest in which to sample the soil, setting the depth of sampling and defining an optimised sampling mission;
- on the basis of the collected samples, the acquired spectra, the "predictive motor" and predictive models implemented on board the drone 10, the system is able to provide, in real time, the chemical/physical values for the soil; the predictive models and motor are determined based on known chemometric analysis techniques.

Some preferred but not mandatory constructional aspects of the drone 10 of the invention are described in the following.

The drone 10 may include a top photovoltaic element 12, which may be axially symmetrical, for example shaped like a nose cone as shown in the accompanying drawings, or in any case cone shaped.

In this case, the battery 13 or other elements powering the propellers of the drone 10 may be housed in a space afforded inside the photovoltaic element 12.

In further detail, the drone 10 may envisage an upper portion, provided with the central frame of the aircraft 10, from which arms extend radially, with the propellers mounted on their ends, and a lower portion comprising a housing 14 for the removable coupling of the spectrometer 4, beneath which the above mentioned sampling device 5 is located.

Between the casing 53 of the sampling device 5 and the walls of the housing 14 an opening is provided to enable the NIR sensors to make measurements of the collected samples 50 of soil.

## Claims

1. An apparatus (1) for the analysis and management of a field (2) cultivated or intended for cultivation, comprising
at least a first detection device (3) mounted on board of a drone (10) and adapted for acquiring electromagnetic radiation emitted by vegetation (21) and/or soil (20) of said field (2) and adapted for producing first measurement signals representative of acquired electromagnetic radiation;
at least a second detection device (4) adapted for spectroscopic analysis of the soil (20) adapted for producing second measurement signals representative of analyses performed;
at least one processing unit (6) comprising:
a first analysis module (61) configured to obtain first data representative of said vegetation (21) and/or said soil (20) of the field (2), as a function of said first measurement signals;
a second analysis module (62) configured to obtain second data of the soil (20) subject to detection, as a function of said second measurement signals; and
an evaluation module configured to determine evaluation states of the soil and vegetation analysed, based on respective first and second data;
**characterized in that** the apparatus (1) includes the drone (10) on which at least a sampling device (5) is mounted, which is adapted for picking one or more samples (50) of soil and comprises a rotating picking auger (51) adapted for removing samples (50) from the soil (20) and at least one collection volume (V) for holding said samples (50);
wherein the second detection device (4) is positioned on board of the drone (10) so as to conduct spectrographic analyses on the samples (50) contained in said volume (V);
wherein the processing unit (6) is adapted for identifying samples (50) corresponding to abnormal values of the respective second data produced by the second analysis module (62);
the apparatus (1) comprising means for retaining said samples **characterised by** abnormal values arranging them in an ordered sequence and the processing unit (6) being configured to store the position of each sample in the sequence, associating the position to relative second data.

2. The apparatus (1) according to the preceding claim, wherein said first analysis module (61) is configured to determine said first data through the processing of images acquired by the first device (3).

3. The apparatus (1) according to at least one of the preceding claims, wherein the first analysis module (61) is configured to determine the first data via thermographic analysis.

4. The apparatus (1) according to at least one of the preceding claims, wherein the first detection device (3) comprises a hyper-spectral camera (30).

5. The apparatus (1) according to at least one of the preceding claims, wherein said second detection device (4) comprises a spectrometer (4) for near-infrared analysis.

6. The machine according to at least one of the preceding claims, wherein said processing unit (6) comprises or is connected to a user interface (64) configured to allow operators to select or set first evaluation parameters representative of the states of vegetation (21) health and/or states of soil (20) organic quality and second evaluation parameters representative of the chemical/physical states of the soil (20), said evaluation module being configured to determine the aforesaid evaluation states as a function of said first and second evaluation parameters, respectively.

7. The apparatus (1) according to at least one of the preceding claims, comprising a receiver for a satellite positioning system, wherein the processing unit (6) comprises a geo-referencing module (63) configured to associate the relative qualitative states determined by the analysis modules (61, 62) to geographical coordinates of field (2) areas examined through said detection devices.

8. The apparatus (1) according to the preceding claim, wherein the processing unit (6) comprises a mapping module (66) configured to produce a map of the field (2) analysed in which the associations created by said geo-referencing module (63) are represented graphically.

9. The apparatus (1) according to claim 8, wherein the drone (10) mounts said receiver.

## Patentansprüche

1. Vorrichtung (1) zur Analyse und Bewirtschaftung eines Feldes (2), das angebaut oder zum Anbau bestimmt ist, umfassend mindestens eine erste Detektionsvorrichtung (3), die an Bord einer Drohne (10) montiert ist und zur Erfassung der von der Vegetation und/oder Boden (20) des Feldes (2) emittierten elektromagnetischen Strahlung (21) ausgelegt ist und zur Erzeugung von ersten Messsignalen, die für die erfasste elektromagnetische Strahlung repräsentativ sind, ausgelegt ist;
mindestens eine zweite Detektionsvorrichtung (4), die zur spektroskopischen Analyse des Bodens (20) ausgelegt ist und die zur Erzeugung von zweiten Messsignalen, die für durchgeführte Analysen repräsentativ sind, ausgelegt ist; mindestens eine Verarbeitungseinheit (6), umfassend:
ein erstes Analysemodul (61), das konfiguriert ist, um erste Daten, die für die Vegetation (21) und/oder den Boden (20) des Feldes (2) repräsentativ sind, als Funktion der ersten Messsignale zu erhalten;
ein zweites Analysemodul (62), das konfiguriert ist, um zweite Daten des Bodens (20), der detektiert werden soll, als Funktion der zweiten Messsignale zu erhalten; und ein Bewertungsmodul, das konfiguriert ist, um Bewertungszustände des analysierten Bodens und der analysierten Vegetation basierend auf den jeweiligen ersten und zweiten Daten zu bestimmen;
**dadurch gekennzeichnet, dass** die Vorrichtung (1) einbezieht
- die Drohne (10), auf der
mindestens eine Probenahmevorrichtung (5) montiert ist, die zum Aufnehmen einer oder mehrerer Bodenproben (50) ausgelegt ist und eine rotierende Aufnahmeschnecke (51), die zum Entfernen von Proben (50) aus dem Boden (20) ausgelegt ist und mindestens eine Sammelvolumen (V) zum Halten der Proben (50) umfasst;
wobei die zweite Detektionsvorrichtung (4) an Bord der Drohne (10) positioniert ist, um spektrographische Analysen an den in dem Volumen (V) enthaltenen Proben (50) durchzuführen;
wobei die Verarbeitungseinheit (6) ausgelegt ist, um Proben (50) zu identifizieren, die abnormalen Werten der jeweiligen zweiten Daten entsprechen, die von dem zweiten Analysemodul (62) erzeugt werden; wobei die Vorrichtung (1) Mittel zum Zurückhalten der Proben umfasst, die durch abnormale Werte gekennzeichnet sind, indem sie sie in einer geordneten Sequenz anordnet, und wobei die Verarbeitungseinheit (6) konfiguriert ist, um die Position jeder Probe in der Sequenz zu speichern, indem die Position relativen zweiten Daten zugeordnet wird.

2. Vorrichtung (1) nach dem vorhergehenden Anspruch, wobei das erste Analysemodul (61) konfiguriert ist, um die ersten Daten durch die Verarbeitung von Bildern zu bestimmen, die von der ersten Vorrichtung (3) erfasst wurden.

3. Vorrichtung (1) nach mindestens einem der vorhergehenden Ansprüche, wobei das erste Analysemodul (61) konfiguriert ist, um die ersten Daten mittels thermographischer Analyse zu bestimmen.

4. Vorrichtung (1) nach mindestens einem der vorhergehenden Ansprüche, wobei die erste Detektionsvorrichtung (3) eine Hyperspektralkamera (30) umfasst.

5. Vorrichtung (1) nach mindestens einem der vorhergehenden Ansprüche, wobei die zweite Detektionsvorrichtung (4) ein Spektrometer (4) zur Nahinfrarotanalyse umfasst.

6. Maschine nach mindestens einem der vorhergehenden Ansprüche, wobei die Verarbeitungseinheit (6) eine Benutzerschnittstelle (64) umfasst oder mit dieser verbunden ist, die konfiguriert ist, um es dem Bediener zu ermöglichen, erste Bewertungsparameter, die für die Gesundheitszustände der Vegetation (21) und/oder Zustände der organischen Qualität des Bodens (20) repräsentativ sind und zweite Bewertungsparameter, die für die chemischen / physikalischen Zustände des Bodens (20) repräsentativ sind, auszuwählen oder einzustellen, wobei das Bewertungsmodul konfiguriert ist, um die Bewertungszustände als Funktion der ersten und zweiten Bewertungsparameter zu bestimmen.

7. Vorrichtung (1) nach mindestens einem der vorhergehenden Ansprüche, umfassend einen Empfänger für ein Satellitenpositionierungssystem, wobei die Verarbeitungseinheit (6) ein Georeferenzierungsmodul (63) umfasst, das konfiguriert ist, um die relativen qualitativen Zustände, die durch die Analysemodule (61, 62) bestimmt werden, zu geografischen Koordinaten von Feldgebieten (2), die durch diese Detektionsvorrichtungen untersucht wurden, zuzuordnen.

8. Vorrichtung (1) nach dem vorhergehenden Anspruch, wobei die Verarbeitungseinheit (6) ein Abbildungsmodul (66) umfasst, das konfiguriert ist, um eine Karte des analysierten Feldes (2) zu erzeugen, in dem die durch das Georeferenzierungsmodul (63) erzeugten Zuordnungen grafisch dargestellt werden.

9. Vorrichtung (1) nach Anspruch 8, wobei die Drohne (10) den Empfänger montiert hat.

## Revendications

1. Appareil (1) pour l'analyse et la gestion d'un champ (2) cultivé ou destiné à être cultivé, comprenant au moins un premier dispositif de détection (3) monté à bord d'un drone (10) et adapté pour acquérir un rayonnement électromagnétique émis par la végétation (21) et/ou le sol (20) dudit champ (2) et adapté pour produire des premiers signaux de mesure représentatifs du rayonnement électromagnétique acquis;
au moins un second dispositif de détection (4) adapté à l'analyse spectroscopique du sol (20) et adapté à la production de seconds signaux de mesure représentatifs des analyses effectuées;
au moins une unité de traitement (6) comprenant:
un premier module d'analyse (61) configuré pour obtenir des premières données représentatives de ladite végétation (21) et/ou dudit sol (20) du champ (2), en fonction desdits premiers signaux de mesure;
un second module d'analyse (62) configuré pour obtenir des secondes données du sol (20) soumis à la détection, en fonction desdits seconds signaux de mesure; et un module d'évaluation configuré pour déterminer des états d'évaluation du sol et de la végétation analysés, sur la base des premières et secondes données respectives;
**caractérisé en ce que** l'appareil (1) inclut le
- drone (10) sur lequel est monté
au moins un dispositif d'échantillonnage (5) étant adapté pour prélever un ou plusieurs échantillons (50) de sol et comprenant une vis sans fin de prélèvement (51) rotative adaptée pour prélever des échantillons (50) du sol (20) et au moins un volume récepteur (V) servant à contenir lesdits échantillons (50);
dans lequel le second dispositif de détection (4) est positionné à bord du drone (10) de manière à effectuer des analyses spectrographiques sur les échantillons (50) contenus dans ledit volume (V);
dans lequel l'unité de traitement (6) est adaptée pour identifier des échantillons (50) correspondant à des valeurs anormales des secondes données respectives produites par le second module d'analyse (62); l'appareil (1) comprenant des moyens servant à retenir lesdits échantillons **caractérisés par** des valeurs anormales en les disposant dans une séquence ordonnée et l'unité de traitement (6) étant configurée pour stocker la position de chaque échantillon dans la séquence, en associant la position à des secondes données relatives.

2. Appareil (1) selon la revendication précédente, dans lequel ledit premier module d'analyse (61) est configuré pour déterminer lesdites premières données à travers le traitement des images acquises par le premier dispositif (3).

3. Appareil (1) selon au moins une des revendications précédentes, dans lequel le premier module d'analyse (61) est configuré pour déterminer les premières données par analyse thermographique.

4. Appareil (1) selon au moins l'une des revendications précédentes,
dans lequel le premier dispositif de détection (3) comprend une caméra hyperspectrale (30).

5. Appareil (1) selon au moins une des revendications précédentes, dans lequel ledit second dispositif de détection (4) comprend un spectromètre (4) pour l'analyse dans l'infrarouge proche.

6. Machine selon au moins une des revendications précédentes, dans laquelle ladite unité de traitement (6) comprend ou est reliée à une interface utilisateur (64) configurée pour permettre aux opérateurs de sélectionner ou de définir des premiers paramètres d'évaluation représentatifs de l'état de santé de la végétation (21) et/ou de l'état de la qualité organique du sol (20) et des seconds paramètres d'évaluation représentatifs de l'état chimique/physique du sol (20), ledit module d'évaluation étant configuré pour déterminer les états d'évaluation susmentionnés en fonction, respectivement, desdits premiers et seconds paramètres d'évaluation.

7. Appareil (1) selon au moins une des revendications précédentes, comprenant un récepteur pour un système de positionnement par satellite, dans lequel l'unité de traitement (6) comprend un module de géoréférencement (63) configuré pour associer les états qualitatifs relatifs déterminés par les modules d'analyse (61, 62) aux coordonnées géographiques des zones du champ (2) examinées par lesdits dispositifs de détection.

8. Appareil (1) selon la revendication précédente, dans lequel l'unité de traitement (6) comprend un module de cartographie (66) configuré pour produire une carte du champ (2) analysé dans laquelle les associations créées par ledit module de géoréférencement (63) sont représentées graphiquement.

9. Appareil (1) selon la revendication 8, dans lequel le drone (10) monte ledit récepteur.
